# EUROPEAN PATENT SPECIFICATION

(11) **EP 2 623 046 B1**
(45) Date of publication and mention of the grant of the patent: **05.08.2015**
(21) Application number: 11828028.8
(22) Date of filing: 24.08.2011
(51) Int. Cl.: A61B 17/16

(54) **DRILL HEAD LOCKING APPARATUS AND DRILL HEAD**
BOHRKOPFVERRIEGELUNGSVORRICHTUNG UND BOHRKOPF
APPAREIL DE VERROUILLAGE À TÊTE D'ALÉSAGE ET TÊTE D'ALÉSAGE

(30) Priority: 30.09.2010 CN 201010298087
(43) Date of publication of application: 07.08.2013
(73) Proprietor: CHONGQING RUNZE PHARMACEUTICAL CO., LTD., Yubei District Chongqing 401120 (CN)
(72) Inventor: YE, Lei, Chongqing 401120 (CN); ZHOU, Jian, Chongqing 401120 (CN); FENG, Hua, Chongqing 401120 (CN); LI, Fei, Chongqing 401120 (CN); ZHU, Hengyang, Chongqing 401120 (CN); LI, Congxiao, Chongqing 401120 (CN)
(74) Representative: Clegg, Richard Ian
(86) International application number: PCT/CN2011/078809
(87) International publication number: WO 2012/041132

(56) References cited:
- CN-A- 101 711 702
- CN-A- 101 926 670
- CN-A- 101 926 671
- CN-A- 101 972 157
- CN-U- 201 492 470
- CN-U- 201 798 776
- CN-U- 201 798 777
- CN-U- 201 798 778
- CN-Y- 201 198 128
- US-A- 5 490 683
- US-B2- 6 890 334

## Description

### Field of the Invention

The present invention relates to a bone drill motor for use in surgery and, more particularly, to a drill head locking apparatus and a drill head thereof.

### Description of the Prior Art

In surgery, the cranial drill, milling cutter and abrasion drill are tools commonly used for craniotomy. The cranial drill comprises a main machine, a reducer, a locking seat and a drill head. The drill head comprises cutting edge in the front part and a transmission rod in the rear part. The drill has a transmission rod drill head, and the locking seat is threadedly connected to the front part of the reducer, having the drill head passing through the locking seat and coupled with the power output of the reducer. The locking seat effects tight locking of the transmission rod to prevent the detachment of the drill head in operation. The prior locking apparatus utilizing a trip to effect locking is inconvenient for mounting and difficult for using.

US 5,490,683 describes apparatus and methods for coupling a driving shaft and a mating tool shaft to transmit rotational forces and axial tension and compression forces between the shafts.

### Summary of the Invention

The object of the present invention is to provide a drill transmission rod locking apparatus and a drill head that are convenient for use.

The object of the present invention is accomplished by adopting the following scheme.

A drill head locking apparatus, wherein the locking apparatus comprises: a flange having a through hole, bearings arranged on the inner wall of a small cylinder on the flange, a locking sleeve arranged over the flange, a protrusion arranged on the upper part of the inner wall of the locking sleeve, the lower part of the inner wall of the locking sleeve having a conical face. A spring is arranged between the bottom face of the conical face and an upper bottom face of the flange. A pressing plate is threadedly connected to the outer wall of the top part of the small cylinder on the upside of the flange and presses against the protrusion. A conical hole is arranged in the corresponding position on the wall of the small cylinder on the upside of the flange and on the conical face, and a steel ball is arranged in the conical hole.

In order to transfer uniform force to the transmission rod, the bearings can be two which are respectively positioned below the outer wall and above the upper bottom face.

In order to ensure the bearings are secured, a supporting member is arranged between the two bearings.

In order to facilitate connecting the reducer, the inner wall of the big cylinder on the underside of the flange is provided with inner threads.

A drill head, mating the noted drill head locking apparatus, comprises a cutting edge in the front part and a transmission rod in the rear part, wherein a groove is provided on the transmission rod.

By adopting this structure, when the transmission rod is to be inserted, one only has to press down the locking sleeve, then the conical face moves down and the steel ball roll toward outside. When the transmission rod reaches the designated position, one releases the locking sleeve which moves up under the effect of the spring, the conical face engages the steel ball and moves inwardly to snap into the snap-slot on the transmission rod which is thus secured. In such a locking manner, the locking and loosening operations are easy and convenient to apply, providing a firm securing effect when in locking status and improved safety.

### Brief Description of the Drawings

Figure 1 is a structural schematic view of a drill head locking apparatus in an embodiment of the present invention;
Figure 2 is a structural schematic view of a drill head in an embodiment of the present invention.

### Detailed Description of the Preferred Embodiments

Hereinafter further description will be made by incorporating figures and embodiments to illustrate the present invention.

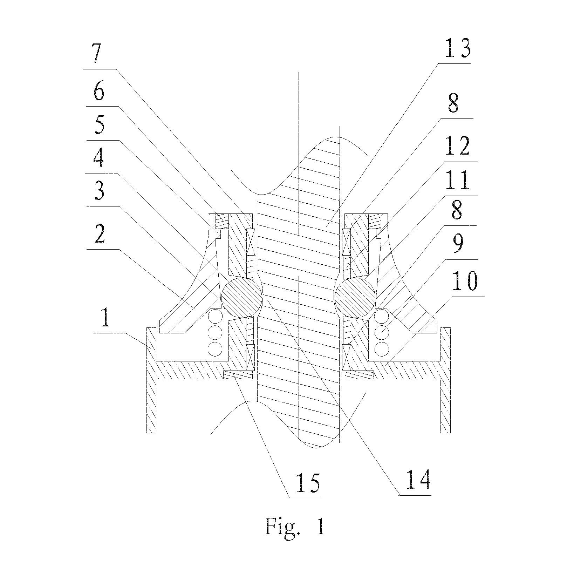

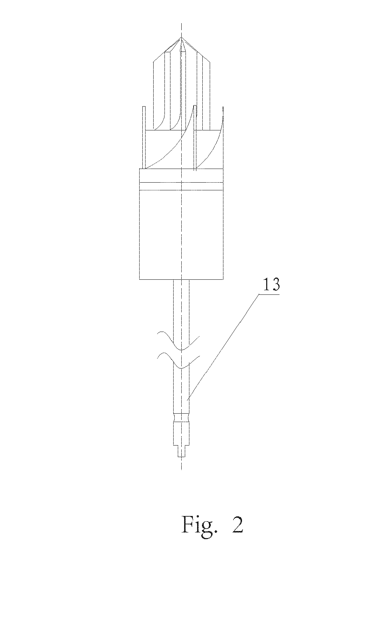

As can be seen from Figure 1 to Figure 2:
A drill head, comprises a cutting edge in the front part and a transmission rod in the rear part, with a groove 14 provided on the transmission rod 13.
A drill head locking apparatus comprises a flange 1 with a through hole. The inner wall of the small cylinder on the upside of the flange is arranged with bearings 8 thereon, which are numbered to be two and respectively positioned below the outer wall 7 and above the top bottom face 10. A supporting member 12 is arranged between the two bearings 8, which may effectively ensure the smooth rotation of the transmission rod to produce lesser heat. A pressing member 15 is threadedly connected to the upper bottom face 10, thereby tightly abutting the bearings 8 and the supporting member 12 and adding to the firmness between the bearings 8 to reduce the likelihood of detachment. A locking sleeve 2 is arranged over the flange, with a protrusion 5 arranged on the upper part of the inner wall of the locking sleeve 2, the lower part of the inner wall of the locking sleeve 2 having a conical face 3. A spring 9 is arranged between the bottom face of the conical face 3 and an upper bottom face 10 of the flange. A pressing plate 6 is threadedly connected on the outer wall 7 of the top part of the small cylinder on the flange and presses against the protrusion 5. A conical hole 11, leading to the through hole of the flange, is arranged on the wall of the small cylinder on the flange, and a steel ball 4 is arranged in the conical hole 11. A groove 14 is provided on the drill transmission rod 13 in the corresponding position to the conical hole 11. The inner wall of the big cylinder on the underside of the flange 1 is provided with inner threads. In this embodiment, the conical holes 11 and the steel balls 4 can sure be numbered to be two, and as a variant, may be set to be three respectively, so that an improved locking effect is offered.

The techniques described herein are exemplary, and should not be construed as implying any particular limitation on the present disclosure. It should be understood that various alternatives, combinations and modifications could be devised by those skilled in the art without departing from the technical scheme content of the present invention. The present disclosure is intended to embrace all alternatives, modifications and variances that fall within the scope of the appended claims.

## Claims

1. A drill head locking apparatus suitable for use in surgery, wherein the apparatus comprises a flange (1) having a through hole, a bearing (8) arranged on the inner wall of a small cylinder on the flange, a locking sleeve (2) arranged over the flange, a protrusion (5) arranged on the upper part of the inner wall of the locking sleeve (2), the lower part of the inner wall of the locking sleeve (2) having a conical face (3); a spring (9) is arranged between the bottom face of the conical face (3) and an upper bottom face (10) of the flange; a pressing plate (6) is threadedly connected to the outer wall (7) of the top part of the small cylinder on the upside of the flange and presses against the protrusion (5); a conical hole (11) is arranged in the corresponding position on the wall of the small cylinder on the upside of the flange and on the conical face (3), and a steel ball (4) is arranged in the conical hole (11).

2. The drill head locking apparatus suitable for use in surgery according to claim 1, **characterized in that**, the bearings (8) are numbered to be two, respectively positioned below the outer wall (7) and above the upper bottom face (10).

3. The drill head locking apparatus suitable for use in surgery according to claim 2, **characterized in that**, a supporting member (12) is arranged between the two bearings (8).

4. The drill head locking apparatus suitable for use in surgery according to any of claims from 1 to 3, **characterized in that**, the inner wall of the big cylinder on the underside of the flange (1) is provided with inner threads.

5. The drill head locking apparatus suitable for use in surgery according to any of claims 1 to 4 mated with a drill head, wherein the drill head comprises a cutting edge in a front part and a transmission rod (13) in a rear part, and a groove (14) is provided on the transmission rod (13).

## Patentansprüche

1. Zur Verwendung in der Chirurgie geeignete Bohrkopfverriegelungsvorrichtung, worin die Vorrichtung einen Flansch (1), der eine Durchgangsbohrung aufweist, ein Lager (8), das an der Innenwand eines kleinen Zylinders auf dem Flansch angeordnet ist, eine Verriegelungshülse (2), die über dem Flansch angeordnet ist, einen Vorsprung (5) umfasst, der am oberen Teil der Innenwand der Verriegelungshülse (2) angeordnet ist, wobei der untere Teil der Innenwand der Verriegelungshülse (2) eine konische Fläche (3) aufweist; wobei eine Feder (9) zwischen der Bodenfläche der konischen Fläche (3) und einer oberen Bodenfläche (10) des Flansches angeordnet ist; wobei eine Druckplatte (6) über ein Gewinde mit der Außenwand (7) des obersten Teils des kleinen Zylinders an der Oberseite des Flansches verbunden ist und gegen den Vorsprung (5) drückt; wobei ein konisches Loch (11) an der entsprechenden Position an der Wand des kleinen Zylinders an der Oberseite des Flansches und auf der konischen Fläche (3) angeordnet ist, und wobei eine Stahlkugel (4) im konischen Loch (11) angeordnet ist.

2. Zur Verwendung in der Chirurgie geeignete Bohrkopfverriegelungsvorrichtung nach Anspruch 1, **dadurch gekennzeichnet, dass** die Lager (8) die Anzahl zwei haben und unterhalb der Außenwand (7) beziehungsweise oberhalb der oberen Bodenfläche (10) angeordnet sind.

3. Zur Verwendung in der Chirurgie geeignete Bohrkopfverriegelungsvorrichtung nach Anspruch 2, **dadurch gekennzeichnet, dass** ein Stützelement (12) zwischen den beiden Lagern (8) angeordnet ist.

4. Zur Verwendung in der Chirurgie geeignete Bohrkopfverriegelungsvorrichtung nach einem der Ansprüche 1 bis 3, **dadurch gekennzeichnet, dass** die Innenwand des großen Zylinders an der Unterseite des Flansches (1) mit einem Innengewinde bereitgestellt ist.

5. Zur Verwendung in der Chirurgie geeignete Bohrkopfverriegelungsvorrichtung nach einem der Ansprüche 1 bis 4, die mit einem Bohrkopf verbunden ist, worin der Bohrkopf in einem vorderen Teil eine Schneidekante und in einem hinteren Teil eine Übertragungsstange (13) umfasst und eine Rille (14) auf der Übertragungsstange (13) bereitgestellt ist.

## Revendications

1. Appareil de verrouillage de tête d'alésage apte à être utilisé en chirurgie, où l'appareil comprend une bride (1) ayant un trou traversant, un palier (8) agencé sur la paroi intérieure d'un petit cylindre sur la bride, un manchon de verrouillage (2) agencé sur la bride, une saillie (5) agencée sur la partie supérieure de la paroi intérieure du manchon de verrouillage (2), la partie inférieure de la paroi intérieure du manchon de verrouillage (2) ayant une face conique (3) ; un ressort (9) est agencé entre la face inférieure de la face conique (3) et une face de fond supérieure (10) de la bride ; une plaque de pression (6) est reliée par filetage à la paroi extérieure (7) de la partie supérieure du petit cylindre sur le côté supérieur de la bride et exerce une pression sur la saillie (5) ; un trou conique (11) est agencé dans la position correspondante sur la paroi du petit cylindre sur le côté supérieur de la bride et sur la face conique (3), et une bille d'acier (4) est agencée dans le trou conique (11).

2. Appareil de verrouillage de tête d'alésage apte à être utilisé en chirurgie selon la revendication 1, **caractérisé en ce que** les paliers (8) sont au nombre de deux, positionnés respectivement en dessous de la paroi extérieure (7) et au-dessus de la face de fond supérieure (10).

3. Appareil de verrouillage de tête d'alésage apte à être utilisé en chirurgie selon la revendication 2, **caractérisé en ce qu'**un élément de support (12) est agencé entre les deux paliers (8).

4. Appareil de verrouillage de tête d'alésage apte à être utilisé en chirurgie selon l'une quelconque des revendications 1 à 3, **caractérisé en ce que** la paroi intérieure du grand cylindre sur le côté inférieur de la bride (1) est pourvue de filetages internes.

5. Appareil de verrouillage de tête d'alésage apte à être utilisé en chirurgie selon l'une quelconque des revendications 1 à 4 apparié à une tête d'alésage, où la tête d'alésage comprend un bord coupant dans une partie frontale et une tige de transmission (13) dans une partie arrière, et une rainure (14) est réalisée sur la tige de transmission (13).
